Europäisches Patentamt

European Patent Office

Office européen des brevets

(11) Veröffentlichungsnummer: **0 339 356**

**A1**

(12) **EUROPÄISCHE PATENTANMELDUNG**

(21) Anmeldenummer: **89106381.0**

(22) Anmeldetag: **11.04.89**

(51) Int. Cl.⁴: **C12M 1/04**

(30) Priorität: **20.04.88 DE 3813194**

(43) Veröffentlichungstag der Anmeldung:
**02.11.89 Patentblatt 89/44**

(84) Benannte Vertragsstaaten:
**AT CH DE FR IT LI SE**

(71) Anmelder: **Fried. Krupp Gesellschaft mit beschränkter Haftung**
**Altendorfer Strasse 103**
**D-4300 Essen 1(DE)**

(72) Erfinder: **Bernstein, Kurt, Dipl.-Ing.**
**Oskar-Graemer-Strasse 11**
**D-4050 Mönchengladbach 2(DE)**
Erfinder: **Houben, Heinz, Prof. Dr.-Ing.**
**Viersener Strasse 377**
**D-4050 Mönchengladbach 1(DE)**
Erfinder: **Zeitsch, Karl-Joachim, Dr.-Ing.**
**Dürener Strasse 393**
**D-5000 Köln 41(DE)**

(54) **Verfahren und Vorrichtung zur Herstellung von Backhefe.**

(57) Die herkömmlichen Verfahren haben einen hohen Energiebedarf. Außerdem ist der "Sauerstoffertrag", das ist die pro Energieeinheit in die Nährlösung eingebrachte Sauerstoffmenge, ausgedrückt in kg $O_2$/kWh, relativ gering. Der Erfindung liegt die Aufgabe zugrunde, den Sauerstoffertrag wesentlich zu steigern, um Hefe-Trockensubstanz mit wesentlich weniger Energie erzeugen zu können als mit herkömmlichen Verfahren.

Diese Aufgabe wird dadurch gelöst, daß die Nährlösung an der Luftzuführungsstelle unter hohen statischen oder dynamischen Druck versetzt und in einem Schlaufenreaktor in erzwungenem Umlauf gehalten wird, wobei die Geschwindigkeit der Nährlösung im begasten Teil wesentlich größer ist als die Steiggeschwindigkeit der Gasblasen relativ zur Nährlösung.

FIG.1

## Verfahren und Vorrichtung zur Herstellung von Backhefe

Die Erfindung bezieht sich auf ein Verfahren zur Hertellung von Backhefe, bei dem einer mit Stellhefe versetzten Nährlösung Luft zugeführt wird, sowie eine Vorrichtung zu seiner Durchführung.

Für die Herstellung von Backhefe werden üblicherweise Fermenter eingesetzt mit einem Höhen-Durchmesser-Verhältnis bis zu 3. Es handelt sich dabei um einen diskontinuierlichen (Batch-) Betrieb, bei welchem der Nährlösung die Luft über ein Belüftungssystem (z.B. Drehlüfter oder Turbinen) am Behälterboden zugeführt wird. Für die Herstellung von 1 t Hefe H27 (Hefe mit einer Trockensubstanz von 27 %, Rest Wasser) aus 1 t Melasse mit einem Zuckergehalt von ca. 50 % liegt die benötigte Luftmenge je nach System zwischen 2000 und 6000 Nm³ pro Tonne Melasse bzw. erzeugter Hefe H27, wobei die kleineren Werte für modernere Systeme (z.B. Turbine) gelten.

Nachteilig bei den bisherigen Herstellungsverfahren ist dieser hohe Luftbedarf wegen des für das Einblasen nontwendigen hohen Bedarfs an elektrischer Energie. Die Kosten für die Herstellung von Backhefe werden somit wesentlich durch den Energiebedarf bestimmt, der zur Einbringung der als Sauerstoffquelle dienenden Luft erforderlich ist. Der Anteil dieses Fermenter-Energiebedarfs macht mindestens 50 % des Gesamtenergiebedarfs in einer Hefefabrik aus. In Anbetracht dieser Tatsache wird die Güte jedes Backhefeprozesses üblicherweise durch den sogenannten "Sauerstoffertrag" charakterisiert. Man versteht darunter die pro Energieeinheit in die Nährlösung eingebrachte Sauerstoffmenge, ausgedrückt in kg $O_2$/kWh. Da der Hefeprozess immer so gesteuert wird, daß praktisch der gesamte in Lösung gebrachte Sauerstoff zur Bildung von Hefe aufgebraucht wird und da die gebildete Hefemenge der aufgebrauchten Sauerstoffmenge proportional ist, ist der genannte "Sauerstoffertrag" proportional dem spezifischen Hefeertrag, d.h. der pro Energieeinheit gebildeten Hefemenge. Aufgrund dieses Zusammenhanges ist der "Sauerstoffertrag" ein direktes Maß für die Wirtschaftlichkeit eines Hefeprozesses. Bei herkömmlichen Hefegeneratoren liegt der "Sauerstoffertrag" bei etwa 1,1 kg $O_2$/kWh, was einem spezifischen Hefeertrag von 1,48 kg Hefetrockensubstanz pro kWh entspricht.

Der Erfindung liegt die Aufgabe zugrunde, den "Sauerstoffertrag" wesentlich zu steigern, so daß sich die Hefetrockensubstanz mit wesentlich weniger Energie erzeugen läßt als mit herkömmlichen Verfahren.

Diese Aufgabe wird dadurch gelöst, daß die Nährlösung an der Luftzuführungsstelle einen hohen statischen oder dynamischen Druck aufweist, vorzugsweise 2 bis 5 bar, daß die Nährlösung in einem aus einer senkrechten, begasten Reaktionsstrecke und einer unbegasten Reaktions-oder Rücklaufstrecke bestehenden Kreislauf so geführt wird, daß die begaste Reaktionsstrecke von unten nach oben durchströmt wird und daß die Nährstoffkonzentration in der senkrechten, begasten Reaktionsstrecke durch Zugabe von Nährlösung an mehreren übereinanderliegenden Stellen eingestellt wird. Bei diesem Verfahren ist es möglich, mit einer gegenüber herkömmlichen Verfahren wesentlich geringeren Luftmenge auszukommen.

Aufgrund der Luftzuführung im unteren Teil der Reaktionsstrecke ergibt sich durch den "Mammutpumpeneffekt" eine erzwungene Konvektion, so daß das Substrat auch ohne mechanische Pumpe im Kreislauf bewegt wird. Die Geschwindigkeit der Flüssigkeit in der begasten Reaktionsstrecke ist dabei wesentlich größer als die Steiggeschwindigkeit der Gasblasen in der Flüssigkeit, d. h. die Relativgeschwindigkeit zwischen Blasen und Flüssigkeit.

In herkömmlichen Hefegeneratoren bewegen sich die Luftblasen durch eine auch Substrat genannte Nährlösung, die praktisch unter Atmosphärendruck steht und entweder keine oder nur eine geringfügige Vertikalbewegung ausführt. Dementsprechend beziehen die oberen Nährlösungsbereiche herkömmlicher Hefegeneratoren ihren Sauerstoff aus Blasen, welche unter einem geringen Druck stehen, was zur Folge hat, daß diese oberen Bereiche nur wenig Sauerstoff erhalten und so die Hefeproduktion limitieren.

Demgegenüber steht bei dem erfindungsgemäßen Verfahren die Nährlösung an der Stelle der Luftinjektion unter einem gegenüber dem Umgebungsdruck stark erhöhten Druck, so daß auch der Sauerstoffpartialdruck in den injizierten Luftblasen entsprechend groß ist, was einen intensiven Sauerstoffübergang von den Blasen in das Substrat gewährleistet. Die Nährlösung bewegt sich dabei mit hoher, erzwungener Geschwindigkeit von unten nach oben, so daß sie nach der unten erfahrenen hohen Sauerstoffbeladung einen großen Teil der letzteren nach oben mitnimmt. Auf diese Weise, d.h. wegen der hohen Sauerstoffbeladung im unteren Bereich und wegen des schnellen Transports der so beladenen Flüssigkeit nach oben, wird über die Höhe der Reaktionsstrecke gesehen im Mittel mehr Sauerstoff eingebracht als in herkömmlichen Prozessen möglich war, und es ist ein wesentliches Merkmal der Erfindung, daß aufgrund des geschilderten Vorgehens der Quotient aus eingebrachtem Sauerstoff und der für dieses Einbringen erforderlichen Energie (kg O₂/kWh) wesentlich über den bisher erreichbaren Werten liegt.

Zur Einstellung einer optimalen Nährstoffkonzentration über die gesamte Höhe der begasten Reaktionsstrecke ist es vorteilhaft, wenn eine Nährstofflösung während des Betriebes an mehreren, übereinanderliegenden und einzeln oder gemeinsam geregelten Nährstoffzuführungen erfolgt. Dadurch ist es möglich, die Zuckerkonzentration über die gesamte Reaktionsstrecke auf einen Wert einzustellen, der eine maximale Hefeproduktion gewährleistet.

Im übrigen ergibt das erfindungsgemäße Verfahren durch seine hohe Umlaufgeschwindigkeit und die daraus resultierende Vermischungsgüte eine besonders gleichmäßige Verteilung sowohl des gelösten Sauerstoffs als auch des zugeführten Nährstoffsubstrats, was eine Äthanolbildung in Teilbereichen verhindert. (Äthanol entsteht einerseits bei einem Unterangebot von Sauerstoff und andererseits bei einem Überangebot von Zucker.) Die vermiedene Äthanolbildung beinhaltet eine Erhöhung der Hefeausbeute pro Nährstoffeinheit. Der daraus resultierende Gewinn durch verminderten Nährstoffbedarf addiert sich zu der durch die bessere Luftausnutzung bedingte Energieersparnis.

Der Vorteil des erfindungsgemäßen Verfahrens besteht in einer Erhöhung des Sauerstoffertrages (kg O₂/kWh), die über 50 % beträgt.

Eine Ausgestaltung entsprechend Anspruch 2, bei der der hohe Druck an der Luftzuführungsstelle durch eine große Höhe der Reaktionsstrecke als statischer Druck aufgebaut wird, hat den Vorteil, daß die längere Verweilzeit der Luftblasen in der Reaktionsstrecke zu einem besseren Stoffaustausch beiträgt.

Eine Alternative des erfindungsgemäßen Verfahrens besteht darin, daß ein vorgebbarer oder an den Verfahrensverlauf anpaßbarer Druck in der Nährlösung durch Drosselung des aus der zugeführten Luft und dem Fermentationsgas bestehenden und oberhalb der begasten Reaktionsstrecke abzuziehenden Gasgemisches eingestellt wird. Es ist somit nicht nur möglich, einen definierten Druck einzustellen, sondern auch den Druck im Verlauf des Prozesses jeweils optimal anzupassen.

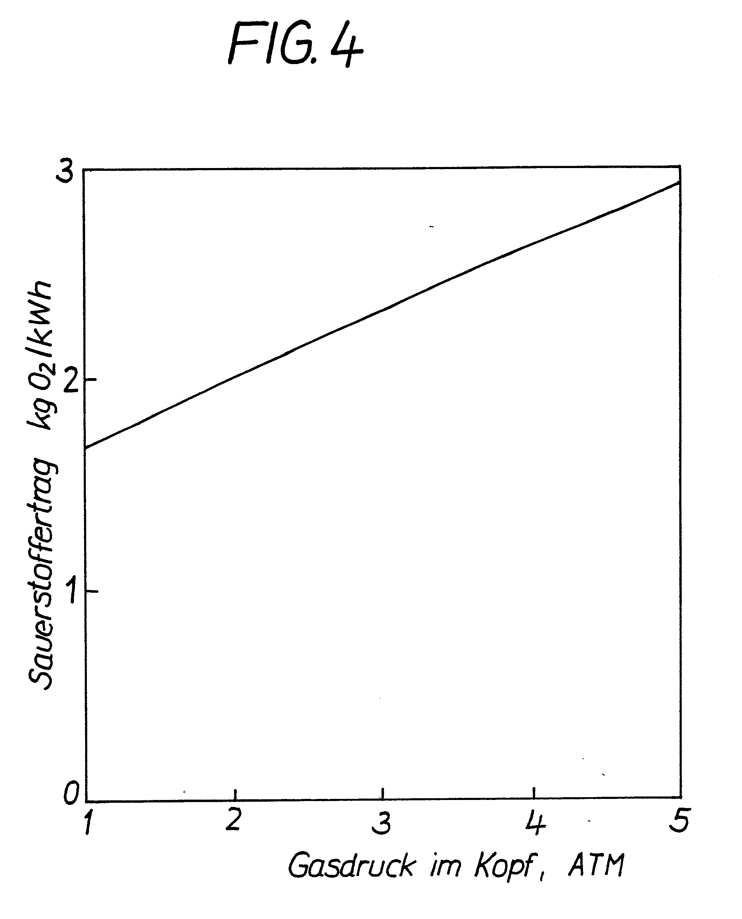

Die durch Erhöhung des Kopfgasdruckes und der damit verbundenen Erhöhung des statischen Druckes an der Eintrittsstelle der Luft erreichbare Verbesserung des "Sauerstoffertrages" ist aus Fig. 4 ersichtlich. In diesem Diagramm ist die Abhängigkeit des "Sauerstoffertrages" vom Kopfgasdruck in bar oder ATM am Beispiel eines Fermenters mit einer Reaktionsstrecke von 14,38 m Höhe dargestellt. Man sieht, daß der oben definierte "Sauerstoffertrag" mit wachsendem Kopfgasdruck beträchtlich ansteigt. Der Grund dafür liegt in der Tatsache, daß die eingebrachte Sauerstoffmenge mit wachsendem Kopfgasdruck stärker zunimmt als die für die Einbringung des Sauerstoffs erforderliche Kompressionsenergie.

Anstelle eines hohen statischen Druckes an der Luftzuführungsstelle kann ein hoher dynamischer Druck durch eine starke Kreisströmung erzeugt werden, indem die aus der unbegasten Rekations- oder Rücklaufstrecke kommende Flüssigkeit mit hoher Geschwindigkeit tangential in einen unten am Reaktor angebrachten Zyklonansatz von kleinem Durchmesser eingespeist wird. Der Flüssigkeitsstrom wird durch eine Pumpe beschleunigt und tritt mit hoher Geschwindigkeit in den Zyklonansatz ein. Die sich dadurch ausbildende intensive Rotationsgeschwindigkeit erzeugt einen hohen dynamischen Druck an der Wand, an der auch die Luftzuführung erfolgt. Die Lufteinspeisung bei hohem dynamischem Druck führt zu der gleichen Erhöhung des "Sauerstoffertrages" wie im Falle eines entsprechenden statischen Druckes (vgl. Fig. 4).

Für die Pumpe bedarf es eines zusätzlichen Energieaufwandes. Dieser Aufwand wird aber durch erhöhte Sauerstoffbeladung der Flüssigkeit mehr als kompensiert, und es läßt sich ein erhöhter "Sauerstoffertrag" ohne große Reaktorhöhe und ohne erhöhten Kopfgasdruck erreichen. Weitere Vorteile dieser Alternative sind, daß sich im Bereich der Luftinjektion die Flüssigkeit senkrecht zur austretenden und senkrecht zur aufsteigenden Luft bewegt, was eine starke Scherung und damit einen schnellen Übergang des Luftsauerstoffs an die Flüssigkeit erlaubt, und daß sich anstelle einer geradlinigen Vertikalbewegung eine Spiralbewegung der Flüssigkeit nach oben ergibt, so daß der Weg der Flüssigkeit und der Gasblasen verlängert wird, was einen verbesserten Stoffaustausch bewirkt.

Je nach verfahrenstechnischen Anforderungen, sowie energietechnischen und räumlichen Gegebenheiten kann es vorteilhaft sein, die erfindungsgemäßen Alternativen zur Erzeugung des Druckes an der Lufteinführungsstelle beliebig zu kombinieren.

Bei einer Ausgestaltung des Verfahrens gemäß Anspruch 6 gelangt die im untenliegenden Injektionsbereich stark mit Sauerstoff gesättigte Nährlösung aufgrund der durch den "Mammutpumpeneffekt" erzwungenen Strömung schnell in den oberen Bereich, was zur Verbesserung des "Sauerstoffertrages" führt. Die Steigbewegung der Luftblasen relativ zur Nährlösung ist dabei gegenüber der erzwungenen Konvektionsströmung wegen der geringen Größe der Blasen sehr klein und ohne Bedeutung. Die Luftzuführung wird so eingestellt, daß in der senkrechten, begasten Reaktionsstrecke eine Geschwindigkeit der flüssigen Phase von 0,5 bis 2 m/s, vorzugsweise 1 m/s vorliegt.

Zur Verbesserung der Sauerstoffbeladung im Injektionsbereich kann es vorteilhaft sein, die Geschwindigkeit der Nährlösung im unteren Teil der Reaktionsstrecke geringer zu halten als im oberen Teil, um damit den Sauerstoffübergang im unteren Teil zu begünstigen. Die mittlere Geschwindigkeit des Substrates wird über die injizierte Luftmenge auf ca. 1 m /s eingestellt.

Eine vorteilhafte Weiterbildung der Erfindung besteht darin, daß die Einspeisung durch mehrere Zyklonansätze von gleichem Drehsinn erfolgt, wobei diese Zyklonansätze vorzugsweise auf einem Kreis um die Achse der begasten Reaktionsstrecke angeordnet sind. Dies bietet sich trotz des damit verbundenen höheren, apparativen Aufwandes für große Reaktoren und vor allem bei größeren Durchmessern an.

Um eine möglichst gleichmäßige und intensive Verteilung der zuzuführenden Luft zu erreichen, ist es vorteilhaft, dem Zyklonansatz oder den Zyklonansätzen die Luft über mehrere am Zyklonumfang verteilte Luftzuführungsstellen zuzuführen und zwar über Injektionskränze, die die Zyklonansätze ringförmig umgeben. Aufgrund der Rotationsströmung ergibt sich ein hoher intensiver dynamischer Druck an der Wand. Dementsprechend muß die über den Injektionskranz eingespeiste Luft eine Flüssigkeitsschicht von hohem Druck passieren, was eine starke Beladung der Flüssigkeit mit Sauerstoff zur Folge hat.

Eine verfahrenstechnisch äußerst vorteilhafte Alternative zur Lufteinspeisung im unteren Teil der Reaktionsstrecke besteht darin, daß der Druck in der Substratlösung in einer zwischen Reaktionsstrecke und Rücklaufstrecke angeordneten Pumpe erhöht wird und die Luftzuführung in einem hinter der Pumpe angeordneten Dispergator erfolgt und das Nährlösung-Luft-Gemisch über ein Überströmventil in die Reaktionsstrecke eingespeist wird. Die für den Hefeprozeß erforderliche Luftmenge wird in den Dispergator, vorzugsweise eine sogenannte Rotor-Stator-Maschine (auch als Homogenisator bezeichnet), unter Druck eingespeist oder von diesem angesaugt und strömt zusammen mit dem Substrat aus der Rücklaufstrecke innerhalb des Dispergators durch eng ineinandergreifende Lochkränze, zwischen denen eine hohe Relativgeschwindigkeit besteht, so daß äußerst kleine Luftbläschen entstehen und diese sehr gleichmäßig verteilt werden, wodurch sich eine extreme Vergrößerung der Grenzfläche zwischen Luft und Nährlösung ergibt. Es wird hierdurch eine praktisch vollkommene Sättigung der Nährlösung mit Sauerstoff erreicht, während im Falle einer konventionellen Einbringung wegen der fehlenden Feinstverteilung der Luft nur eine unvollkommene Annäherung an die Sättigung möglich ist.

Der Druck, unter dem die Luft im Dispergator in die Nährlösung eingebracht wird, kann unabhängig vom Druck in der Reaktionsstrecke durch Drosselung z.B. in einem Überströmventil eingestellt werden. Damit ist es möglich, die Sauerstoffanreicherung der Nährlösung am Eintritt in die begaste Reaktionsstrecke frei wählbar und unabhängig von der Höhe der Reaktionsstrecke einzustellen, indem durch das Überströmventil der Druck im Dispergator so eingestellt wird, daß er wesentlich höher ist als der statische Druck in der begasten Reaktionsstrecke. Dementsprechend wird die Flüssigkeit sehr stark mit Sauerstoff beladen und kann sogar nach Passieren des Ventils bei dem im Unterteil des Reaktors herrschenden geringeren Druck eine beträchtliche Übersättigung mit Sauerstoff aufweisen.

Natürlich ist bei dieser Alternative für den Dispergator und eine in Strömungsrichtung davor anzuordnende Pumpe ein zusätzlicher Energieaufwand notwendig. Bei geeigneter Wahl dieser Aggregate ergibt sich aber dennoch aufgrund der erhöhten Sauerstoffnutzung für die pro Gewichtseinheit Hefetrockensubstanz aufzuwendende Energie ein beachtlicher Verfahrensvorteil.

Bei diskontinuierlicher Arbeitsweise wird das erfindungsgmäße Verfahren ggf. mit an die steigende Hefemenge angepaßten Drücken bis zur Erschöpfung der Nährlösung fortgesetzt und schließlich die Hefe abgezogen.

Das Verfahren nach der Erfindung ist aber auch mit großem Vorteil für eine kontinuierliche Hefeproduktion einzusetzen und erleichtert eine optimale Prozeßführung durch die Einstellbarkeit des Druckes. Dies gilt für ein Verfahren sowohl mit oder ohne Dispergator, d.h. bei statischem Druck an der Luftzuführungsstelle als auch für das Verfahren mit Zyklonansatz d.h. bei dynamischem Druck.

Zur Durchführung eines Verfahrens der vorstehend beschriebenen Art ist eine Vorrichtung nach Anspruch 11 vorgesehen. Es handelt sich hierbei um einen stehenden Behälter mit Füllöffnung bzw. Zuführung für die Stellhefe und die Nährlösung oben sowie einen Hefeabzug unten, der die begaste Reaktionsstrecke beinhaltet. Die unbegaste Reaktions- bzw. Rücklaufstrecke ist vorzugsweise als rohrförmige, außenliegende Rücklaufschleife ausgebildet, die an dem eigentlichen Reaktionsgefäß im oberen und unteren Bereich angeschlossen ist. Die unbegaste Reaktionsstrecke kann jedoch auch aus mehreren außenliegenden Rohren bestehen. Im unteren Bereich des stehenden Behälters ist eine Luftverteileinrichtung mit einem Lochsystem aus sehr vielen, kleinen Bohrungen angeordnet, die über eine seitlich oder unterhalb des Behälters angeordnete Luftzuführung mit Druckluft versorgt wird. Der sich dadurch ergebende Mammutpumpeneffekt bringt den Generatorinhalt in Pfeilrichtung in Umlauf. Der unverbrauchte Luftanteil und das bei der Hefebildung entstandene Kohlendioxid werden am Kopf des Apparates abgeführt.

Zur Einstellung einer optimalen Nährstoffkonzentration während des Betriebes über die gesamte Höhe der begasten Reaktionsstrecke weist der Hefegenerator mehrere, übereinanderliegende Nährstoffzuführungen auf, die einzeln oder gemeinsam geregelt werden können.

Zur Erzeugung eines hohen statischen Druckes kann der Fermenter mit einer großen Bauhöhe ausgeführt werden, die eine Höhe der Reaktionsstrecke von über 10 m, vorzugsweise 15 bis 20 m, ergibt. Ein solcher turmartiger Hefegenerator ist entgegen der herrschenden Meinung der Fachwelt, daß eine hohe Reaktionsstrecke wegen der steigenden Gebläseleistung unwirtschaftlich sei, wesentlich höher als die üblichen Ausführungen. Die Höhe der begasten Reaktionsstrecke beträgt über 10 m. Es konnte nämlich gefunden werden, daß die Vorteile eines hohen statischen Druckes an der Einspeisestelle der Luft die Nachteile einer durch die höhere Ausführung bedingten, höheren Gebläseleistung überwiegen. Eine günstige Relation zwischen Investitionskosten und Ausbeuteverbesserung ergibt sich bei einer Höhe der begasten Reaktionsstrecke von 15 bis 20 m. Die Druckluft wird im unteren Bereich über eine Luftverteileinrichtung in den Hefegenerator eingespeist.

Alternativ zum Aufbau eines hohen statischen Druckes kann es vorteilhaft sein, an der Luftzuführungsstelle einen hohen dynamischen Druck zu erzeugen. Dazu ist an einem senkrecht stehenden, als Schlaufenreaktor mit mehreren übereinanderliegenden Nährstoffzuführstellen ausgebildeten Fermenter am unteren Ende mindestens ein im Verhältnis zum Fermenterdurchmesser kleiner Zyklonansatz mit Luftzuführung und tangentialem Einlaßstutzen für die aus der unbegasten Reaktions- oder Rücklaufstrecke kommende Flüssigkeit angeordnet. Es kann ausreichend für einen hohen Stoffaustausch sein, lediglich einen Zyklonansatz zentral zur Achse der begasten Reaktionsstrecke bzw. des Fermenters anzuordnen. Dies bedeutet einen relativ geringen baulichen Aufwand, da bei einem solchen Fermenter dem Mehraufwand für den Zyklon eine Einsparung an Bauhöhe des Fermenters gegenübersteht. In anderen Fällen kann es, insbesondere bei Fermentern mit größerem Durchmesser vorteilhaft bzw. erforderlich sein, anstelle eines zentralen Zyklonansatzes mehrere Zyklonansätze am unteren Ende des Fermenters anzuordnen und zwar vorzugsweise auf einem Kreis um die Achse der Reaktionsstrecke bzw. des Fermenters. Gegenüber einem einzelnen Zyklon bedeutet dies zwar einen höheren baulichen Aufwand, hat jedoch verfahrenstechnische Vorteile, die diesen Aufwand rechtfertigen können. Zur Luftzuführung ist jeder Zyklonansatz vorzugsweise mit einem ringförmigen Injektionskranz versehen. Vorteilhaft ist es, wenn zur Beschleunigung der Rücklaufflüssigkeit und gleichzeitigen Druckerhöhung jeweils eine Kreiselpumpe zwischen unbegaster Rücklaufstrecke und tangentialem Einlaufstutzen angeordnet ist.

Nach einer Weiterbildung der erfindungsgemäßen Vorrichtung ist vorgesehen, daß die Strömungsquerschnitte in der unbegasten Reaktionsstrecke ggf. durch Anordnung einer Blende so bemessen sind, daß sich in der begasten Reaktionsstrecke eine Geschwindigkeit zwischen 0,5 und 2 m/s ergibt. Das ist dann auch annähernd die Geschwindigkeit der Gasblasen relativ zur Behälterwand, denn in Anbetracht der geringen Größe der Blasen ist ihre Steigbewegung relativ zur Flüssigkeit im Verhältnis zur Flüssigkeitsgeschwindigkeit unbedeutend klein.

Um eine Verbesserung der Sauerstoffbeladung der Suspension zu erreichen, kann nach einer Weiterbildung der Erfindung der Durchmesser des Hefegenerators im Injektionsbereich größer ausgeführt sein als oben. Die Durchmesserabnahme von unten nach oben ist vorzugsweise kontinuierlich und linear.

Zur Erhöhung des statischen Druckes im Bereich der Lufteinspeisung über den geodätischen Druck hinaus ist es vorteilhaft, zur Einstellung eines entsprechenden Kopfgasdruckes am Kopfende des Hefegenerators eine regelbare Drossel anzuordnen, über die das Kopfgasgemisch abgezogen wird.

Zur Verbesserung des Sauerstoffüberganges kann es nützlich sein, die Luft in sehr kleinen Bläschen und in sehr feiner Verteilung in die rücklaufende Suspension einzutragen. Dazu dient eine Vorrichtung nach Anspruch 20. In dem Dispergator wird mit Hilfe der vorgeschalteten Pumpe und des nachgeschalteten Ventils, das vorzugsweise als Überströmventil ausgeführt ist, ein Druck gehalten, der wesentlich über dem statischen Druck am unteren Ende der begasten Reaktionsstrecke liegt. Als Dispergator eignet sich ganz besonders eine Rotor-Stator-Maschine ähnlich den in den Schriften DE 30 09 233 C2, DE 33 42 304 A1 und EPO 253 139 A1 beschriebenen, in einer Ausführung mit Düsenwerkzeug. Mit einer solchen Rotor-Stator-Maschine lassen sich im Scherfeld der Werkzeuge Gasbläschen herunter bis in die Größenordnung von 1 μ erzeugen und gleichmäßig in die Rücklaufsuspension verteilen. Besonders kostengünstig ist eine Ausführung dieser erfindungsgemäßen Ausführungsform, bei der die Verteileinrichtung aus einem einfachen Lochsystem besteht mit relativ großen, leicht einzubringenden, nach oben weisenden Bohrungen, z.B. in einem Rohrbogen oder einer Rohrschlange.

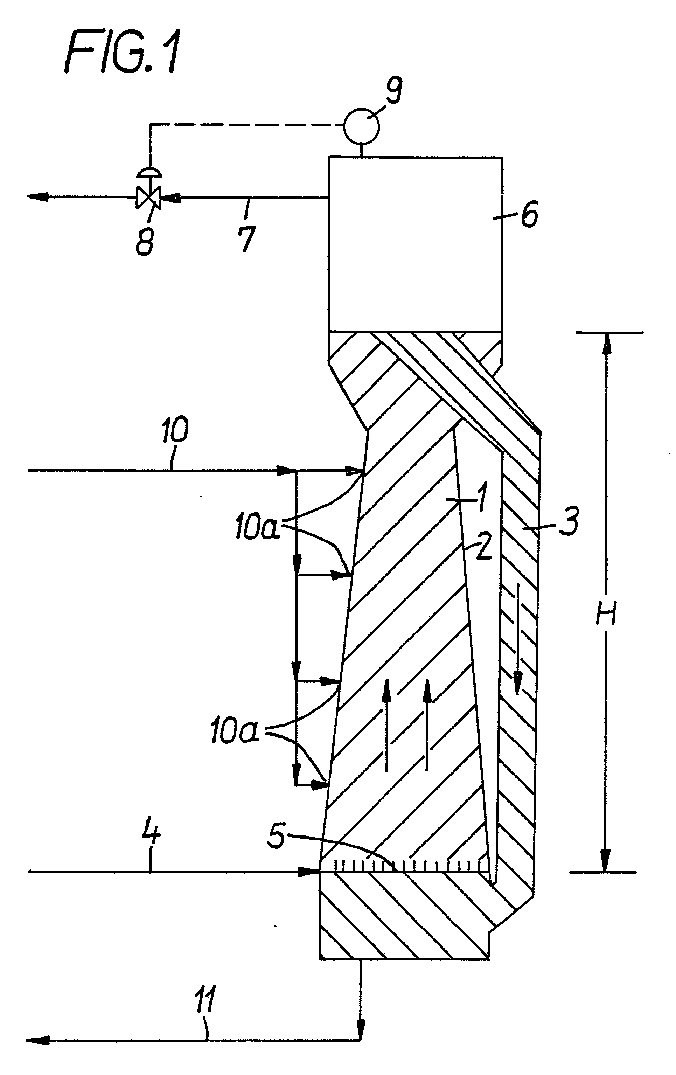

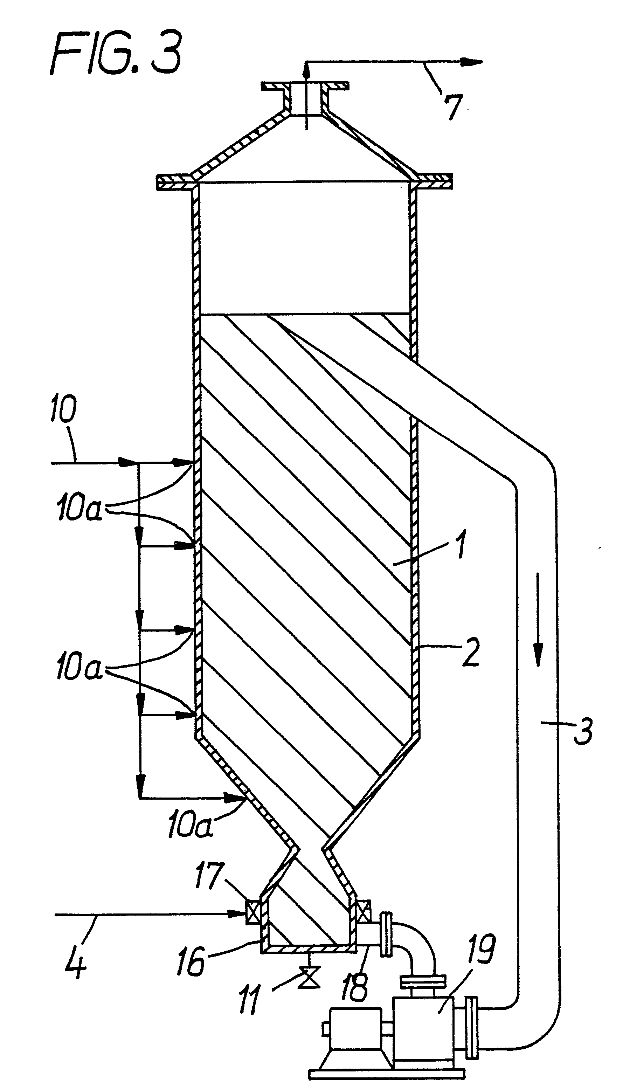

Drei Ausführungsmöglichkeiten der Erfindung sind in den Fig. 1 und 3 schematisch dargestellt und nachfolgend näher beschrieben, wobei auf die Darstellung von Details, die dem Fachmann geläufig sind, verzichtet wurde. Es zeigen

Fig. 1 die Verfahrensführung und den Schlaufenreaktor im Längsschnitt schematisch mit Ventil zur Regelung des Kopfgasdruckes,

Fig. 2 eine Anordnung mit Dispergator in der Rückführung und

Fig. 3 einen Fermenter mit Zyklonansatz.

Dargestellt in Fig. 1 ist eine Ausführungsform, bei der die begaste Reaktionsstrecke 1 des als Schlaufenreaktor ausgebildeten Fermenters im wesentlichen aus einem langen, schlanken, sich vorzugsweise von unten nach oben verjüngenden Behältermantel 2 besteht. Die Höhe der Reaktionsstrecke ist mit H bezeichnet. Die außenliegende, unbegaste, auch Rücklauf genannte Reaktionsstrecke 3 besteht aus einer oder mehreren Rohren, die mit der begasten Reaktionsstrecke 1 oben und unten verbunden ist. Im unteren Teil des Schlaufenreaktors im Bereich des Rücklaufeintritts in die begaste Reaktionsstrecke 1 aber oberhalb des unteren Anschlusses der unbegasten Reaktionsstrecke 3 ist die Luftzuführung 4 angeordnet, wobei die eintretende Luft über eine Luftverteileinrichtung 5 in kleine Luftblasen gleichmäßig über den Querschnitt der begasten Reaktionsstrecke 1 verteilt wird. Das bei der Hefeproduktion entstehende $CO_2$ wird zusammen mit der Restluft über den Fermentergas-Abzug 7 am oberen Ende des Schlaufenreaktors abgeführt. Um einen vorgegebenen Druck im Kopf 6 des Fermenters aufrechtzuerhalten, liegt im Fermentergas-Abzug 7 ein Ventil 8, das über den Druckgeber 9 geregelt wird. Der Rücklauf 3 ist in diesem Falle so dimensioniert, daß sich aufgrund der durch diese Abmessungen und der über die Luftzuführung 4 zugeführten Luftmenge eine vorgegebene, aufwärtsgerichtete Geschwindigkeit in der begasten Reaktionsstrecke 1 ergibt. Über die Substratzuführung 10 wird an mehreren übereinanderliegenden Zuführstellen 10a während des Prozesses - abhängig von der Hefekonzentration geregelt - Substrat bzw. Nährlösung zugeführt. Das Substrat mit der sich bildenden Hefe steigt in der begasten Reaktionsstrecke 1 auf und fließt über die unbegaste Reaktionsstrecke 3 zurück. Die Hefe wird am Ende des Prozesses über den Hefeabzug 11 am unteren Ende aus dem Schlaufenreaktor abgezogen.

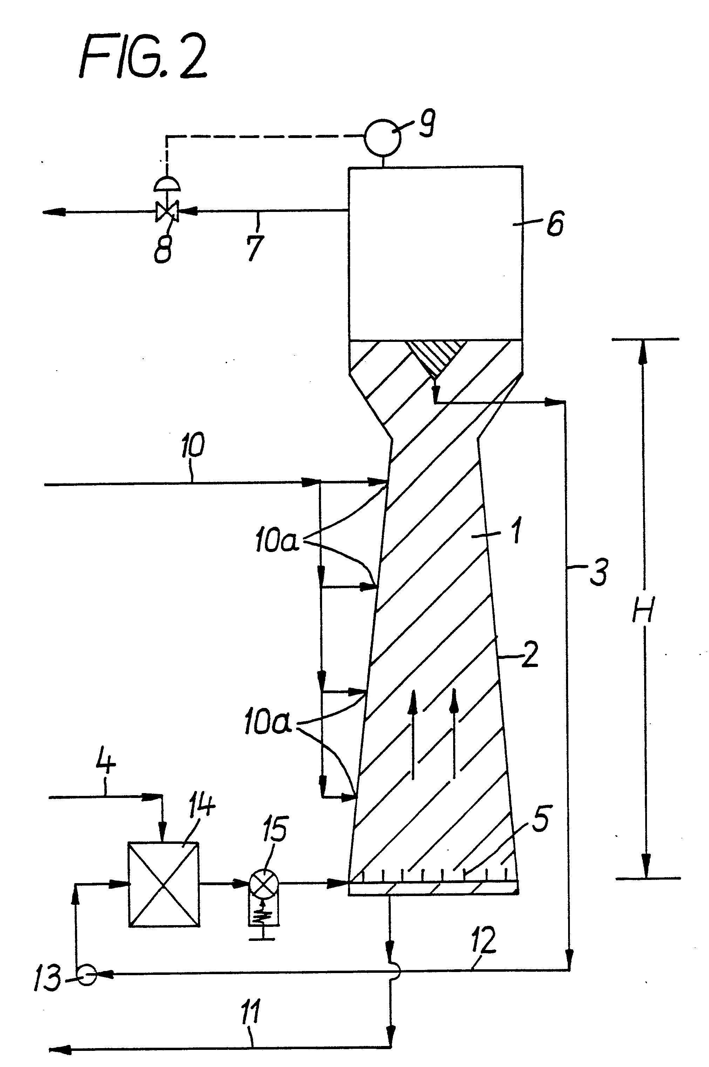

Die in Fig. 2 dargestellte Verfahrensführung unterscheidet sich in bezug auf die Rückführung des Substrats und die Luftzuführung von der in Fig. 1 dargestellten. Das Substrat aus der unbegasten Reaktionsstrecke 3 wird in diesem Falle nicht unmittelbar in den Fuß des Hefegenerators eingeleitet. Es wird über eine Rücklaufleitung 12 einer Pumpe 13 zugeführt. Nach Druckerhöhung in der Pumpe 13 gelangt das Substrat in einen Dispergator 14, bei dem es sich vorzugsweise um eine Rotor-Stator-Maschine mit Düsenwerkzeug handelt. Die für den Hefeprozeß erforderliche Luft wird in den Dispergator 14 eingespeist oder von diesem angesaugt und strömt zusammen mit dem Reaktorrücklauf innerhalb des Dispergators 14 durch eng ineinandergreifende Lochkränze, zwischen denen eine hohe Relativgeschwindigkeit besteht, so daß äußerst kleine Luftbläschen mit insgesamt extrem großer Oberfläche entstehen, die einen spontanen Stoffaustausch zwischen Luft und Flüssigkeit gewährleisten. Mit Hilfe des Ventils 15, das als Überströmventil ausgebildet ist, wird im Dispergator 14 ein Druck gehalten, der wesentlich über dem statischen Druck im unteren Bereich der begasten Reaktionsstrecke 1 liegt. Dementsprechend wird die Flüssigkeit sehr stark mit Sauerstoff beladen und kann sogar nach Passieren des Ventils 15 bei dem im Unterteil des Reaktors bzw. am Eintritt in die Reaktionsstrecke 1 herrschenden geringeren Druck eine beträchtliche Übersättigung mit Sauerstoff aufweisen.

In Fig. 3 ist ein Schlaufenreaktor mit einer begasten Reaktionsstrecke 1, einem Behältermantel 2, einer unbegasten Reaktions- oder Rücklaufstrecke 3, einer Luftzuführung 4, einem Fermentergasabzug 7, einer Substratzuführung 10 mit mehreren, übereinanderliegenden Zuführstellen 10a sowie einem Hefeabzug 11 dargestellt. Dieser Schlaufenreaktor weist unten einen Zyklonansatz 16 mit einem im Verhältnis zum Behältermantel 2 kleinen Durchmesser auf. Der Zyklonansatz 16 ist von einem Injektionskranz 17 zur Lufteinspeisung umgeben und hat einen tangentialen Einlaßstutzen 18. Der Flüssigkeitsstrom aus dem Rücklauf 3 wird durch eine Pumpe 19 beschleunigt und tritt mit hoher Geschwindigkeit durch den Einlaßstutzen 18 in den Zyklonansatz 16 ein, so daß sich in diesem eine intensive Rotationsströmung ergibt, die einen hohen dynamischen Druck an der Wand erzeugt.

**Ansprüche**

1. Verfahren zur Herstellung von Backhefe, bei dem einer mit Stellhefe versetzten Nährlösung Luft zugeführt wird,
**dadurch gekennzeichnet,**
daß die Nährlösung an der Luftzuführungsstelle einen hohen statischen oder dynamischen Druck aufweist, vorzugsweise 2 bis 5 bar, daß die Nährlösung in einem aus einer senkrechten, begasten Reaktionsstrecke (1) und einer unbegasten Reaktions- oder Rücklaufstrecke (3) bestehenden Kreislauf so geführt wird, daß die begaste Reaktionsstrecke (1) von unten nach oben durchströmt wird und daß die Nährstoffkonzentration in der senkrechten, begasten Reaktionsstrecke (1) durch Zugabe von Nährlösung an mehreren übereinanderliegenden Stellen (10a) eingestellt wird.

2. Verfahren nach Anspruch 1, dadurch gekennzeichnet, daß der hohe Druck an der Luftzuführungsstelle durch eine große Höhe (H) der Reaktionsstrecken (1 und 3) erreicht wird, wobei diese Höhe mindestens 10 m, vorzugsweise 15 bis 20 m, beträgt.

3. Verfahren nach Anspruch 1, dadurch gekennzeichnet, daß ein vorgebbarer oder an den Verfahrensverlauf anpaßbarer Druck in der Nährlösung durch Drosselung des aus der zugeführten Luft und dem Fermentationsgas bestehenden und oberhalb der begasten Reaktionsstrecke (1) abzuziehenden Gasgemisches eingestellt wird.

4. Verfahren nach Anspruch 1, dadurch gekennzeichnet, daß der hohe Druck an der Luftzuführungsstelle durch Erzeugung einer starken Kreisströmung erzeugt wird, indem die aus der unbegasten Reaktions- oder Rücklaufstrecke (3) kommende Flüssigkeit mit hoher Geschwindigkeit tangential in mindestens einen unten am Reaktor angebrachten Zyklonansatz (16) eingespeist wird.

5. Verfahren nach Anspruch 1, dadurch gekennzeichnet, daß der hohe Druck an der Lufteinführungsstelle durch eine beliebige Kombination der Maßnahmen entsprechend den Ansprüchen 2 bis 4 erzeugt wird.

6. Verfahren nach einem der Ansprüche 1 bis 5, dadurch gekennzeichnet, daß die Luftzuführung so eingestellt wird, daß in der senkrechten, begasten Reaktionsstrecke (1) eine Geschwindigkeit der flüssigen Phase von 0,5 bis 2 m/s vorzugsweise 1 m/s, vorliegt.

7. Verfahren nach einem der Ansprüche 1 bis 6, dadurch gekennzeichnet, daß die Geschwindigkeit der aufwärtsgerichteten Strömung der Nährlösung im unteren Teil der begasten Reaktionsstrecke (1) geringer ist als im oberen Teil.

8. Verfahren nach Anspruch 4, dadurch gekennzeichnet, daß die Einspeisung durch mehrere Zyklonansätze (16) von gleichem Drehsinn erfolgt, wobei diese Zyklonansätze (16) vorzugsweise auf einem Kreis um die Achse der begasten Reaktionsstrecke (1) angeordnet sind.

9. Verfahren nach Anspruch 4 oder 8, dadurch gekennzeichnet, daß die Lufteinspeisung durch den oder die Zyklonansätze (16) jeweils ringförmig umgebende Injektionskränze (17) erfolgt.

10. Verfahren nach einem der Ansprüche 1 bis 3, dadurch gekennzeichnet, daß die Luftzuführung (4) in einem zwischen begaster Reaktionsstrecke (1) und unbegaster Reaktions- bzw. Rücklaufstrecke (3) angeordneten Dispergator (14) erfolgt und das Nährlösung-Luft-Gemisch über ein Ventil (15) mittels Verteilereinrichtung (5) in die begaste Reaktionsstrecke (1) eingespeist wird.

11. Vorrichtung zur Durchführung eines Verfahrens nach einem der Ansprüche 1, 2, 3, 5, 6, 7 oder 10, dadurch gekennzeichnet, daß ein senkrecht stehender Fermenter mit Hefeabzug (11) und Luftzuführung (4) als Schlaufenreaktor mit einer begasten Reaktionsstrecke (1) und einer unbegasten Reaktions- oder Rücklaufstrecke (3) ausgebildet ist und mehrere übereinanderliegende Nährstoffzuführungsstellen (10a) sowie im unteren Bereich des Fermenters eine Luftverteileinrichtung (5) aufweist.

12. Vorrichtung zur Durchführung eines Verfahrens nach einem der Ansprüche 4, 8 oder 9, dadurch gekennzeichnet, daß ein senkrecht stehender Fermenter mit Hefeabzug (11) als Schlaufenreaktor mit einer begasten Reaktionsstrecke (1) und einer unbegasten Reaktions- oder Rücklaufstrecke (3) ausgebildet ist und mehrere übereinanderliegende Nährstoffzuführstellen (10a) sowie an seinem unteren Ende mindestens einen Zyklonansatz (16) mit Luftzuführung (4) und tangentialem Einlaßstutzen (18) aufweist.

13. Vorrichtung nach Anspruch 12, dadurch gekennzeichnet, daß nur ein Zyklonansatz (16) vorgesehen ist und zwar zentral zur Achse der begasten Reaktionsstrecke (1).

14. Vorrichtung nach Anspruch 12, dadurch gekennzeichnet, daß mehrere Zyklonansätze (16) am unteren Ende des Fermenters angeordnet sind und zwar vorzugsweise auf einem Kreis um dessen Achse.

15. Vorrichtung nach einem der Ansprüche 12 bis 14, dadurch gekennzeichnet, daß der oder die Zyklonansätze (16) jeweils ringförmig von einem Injektionskranz (17) umgeben sind.

16. Vorrichtung nach einem der Ansprüche 12 bis 15, dadurch gekennzeichnet, daß zwischen der unbegasten Rücklaufstrecke (3) und dem tangentialen Einlaufstutzen (18) jeweils eine Pumpe (19) mit Motor (20) angeordnet ist.

17. Vorrichtung nach einem der Ansprüche 11 bis 16, dadurch gekennzeichnet, daß die Strömungsquerschnitte in der unbegasten Reaktionsstrecke (3) gegebenenfalls durch Anordnung einer Blende so bemessen sind, daß in der begasten Reaktionsstrecke (1) eine Geschwindigkeit von 0,5 bis 2 m/s einstellbar ist.

18. Vorrichtung nach einem der Ansprüche 11 bis 17, dadurch gekennzeichnet, daß die begaste Reaktionsstrecke (1) konisch ausgebildet ist mit dem größeren Durchmesser am unteren Ende.

19. Vorrichtung nach einem der Ansprüche 11 bis 18, dadurch gekennzeichnet, daß am Kopfende eine regelbare Drossel (8) angeordnet ist.

20. Vorrichtung nach einem der Ansprüche 12, 17, 18 oder 19 dadurch gekennzeichnet, daß die unbegaste Reaktionsstrecke (3) als außerhalb des die begaste Reaktionsstrecke (1) bildenden Behältermantels (2) verlaufendes Rohr ausgebildet ist und sich eine Rückführleitung (12), eine Pumpe (13) ein Dispergator (14) mit Luftzuführung (4) und ein Ventil (15) zur Druckeinstellung anschließt, das seinerseits auslaßseitig mit der Luftverteileinrichtung (5) verbunden ist.

21. Vorrichtung nach Anspruch 20, dadurch gekennzeichnet, daß der Dispergator (14) eine Rotor-Stator-Maschine mit Düsenwerkzeug ist.

22. Vorrichtung nach Anspruch 20 oder 21, dadurch gekennzeichnet, daß die Luftverteileinrichtung (5) aus einem einfachen Lochsystem mit relativ großen Bohrungen besteht.

# FIG.1

EVA-1/8

# FIG.2

EV 47/8

# FIG. 3

EV 41/86

# FIG. 4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | US-A-4 282 328  (H. FUKUDA et al.)<br>* Figuren; Ansprüche * | 1,11,18 | C 12 M    1/04 |
| Y | | 1-3,5-7,11,18 | |
| | --- | | |
| X | FR-A-2 503 181  (VSESOJUZNY NAUCHNO-ISSLEDOVATELSKY INSTITUT BIOSINTEZA BELKOVYKH VESCHESTV)<br>* Figuren; Ansprüche * | 1,11 | |
| | --- | | |
| A | BE-A-  625 493  (A.P.V. CO., LTD)<br>* Figur; Ansprüche * | 1,11 | |
| | --- | | |
| X | FR-A-2 376 898  (J.H. SCHICK)<br>* Ansprüche; Figuren; Beispiele; Seite 8, Zeile 13 - Seite 9, Zeile 24 * | 1,4,9-12 | |
| | --- | | |
| Y | FR-A-2 229 451  (I.C.I.)<br>* Figuren; Anspruch 1; Seite 16; Seite 10, Zeile 35 * | 1-3,5-7,11,18 | |
| | --- | | RECHERCHIERTE SACHGEBIETE (Int. Cl.4) |
| A | DE-A-2 714 708  (I.C.I.)<br>* Anspruch * | 1 | C 12 M<br>C 12 P |
| | --- | | |
| A | BIOTECHNOLOGY AND BIOENGINEERING, Band 20, 1978, Seiten 1393-1406, John Wiley & Sons, Inc.; M. SERIEYS et al.: "Design and oxygen-transfer potential of a pulsed continuous tubular fermentor"<br>* Figur 1; Seiten 1394-1395 * | 1,11 | |
| | ----- | | |

Der vorliegende Recherchenbericht wurde für alle Patentansprüche erstellt

| Recherchenort | Abschlußdatum der Recherche | Prüfer |
|---|---|---|
| DEN HAAG | 03-08-1989 | COUCKE A.O.M. |